# EUROPÄISCHE PATENTSCHRIFT

(11) **EP 0 549 605 B1**
(45) Veröffentlichungstag und Bekanntmachung des Hinweises auf die Patenterteilung: **04.01.1995**
(21) Anmeldenummer: 91914834.6
(22) Anmeldetag: 22.08.1991
(51) Int. Cl.: A61M 15/00

(54) **TREIBGASFREIES INHALATIONSGERÄT MIT FREMDLUFTSTROM**
INHALATOR WITHOUT PROPELLER GAS WITH FOREIGN AIR STREAM
INHALATEUR SANS GAZ PROPULSEUR A COURANT D'AIR EXTERIEUR

(30) Priorität: 30.08.1990 DE 4027391
(43) Veröffentlichungstag der Anmeldung: 07.07.1993
(73) Patentinhaber: BOEHRINGER INGELHEIM INTERNATIONAL GmbH, 55218 Ingelheim am Rhein (DE); BOEHRINGER INGELHEIM KG, 55216 Ingelheim (DE)
(72) Erfinder: POSS, Gerhard, D-6905 Schriesheim (DE); WITTEKIND, Jürgen, D-6000 Frankfurt/Main 70 (DE); KÜHNEL, Andreas, D-6370 Oberursel 6 (DE)
(74) Vertreter: Fuchs Mehler Weiss
(86) Internationale Anmeldenummer: EP9101593
(87) Internationale Veröffentlichungsnummer: WO9204068

(56) Entgegenhaltungen:
- EP-A- 0 166 294
- WO-A-90/07351
- FR-A- 2 238 505
- FR-A- 2 598 918
- GB-A- 2 144 997
- US-A- 2 587 215
- US-A- 3 900 138
- US-A- 4 274 403

## Beschreibung

Die Erfindung bezieht sich auf ein treibgasfreies Inhalationsgerät mit einem Vorratsraum für eine zu inhalierende, pulverförmige medizinische Substanz, ihm zugeordnet eine manuell betätigbare Dosiereinrichtung zur Aufnahme einer vorgegebenen Dosis der medizinischen Substanz für den jeweiligen Inhalationsvorgang in mindestens einer Dosierkammer, und mit einem seitlichen Mundstück zum aktiven Einatmen, welches einen Luftkanal zur Verteilung der jeweiligen Dosis der medizinischen Substanz im Luftstrom aufweist.

Ein derartiges Inhalationsgerät ist aus der DE 35 35 561 A1 bekannt geworden.

Bei diesem bekannten Inhalationsgerät ist unterhalb dem Vorratsbehälter und parallel zum Mundstück eine Drehschleuse vorgesehen, die Vertiefungen (Dosierkammern) zum Bemessen der medizinischen Substanz aufweist. Sind die Vertiefungen jeweils dem Vorratsbehälter zugewendet, werden sie selbsttätig befüllt. Wird durch eine 180-Grad-Verdrehung der Drehschleuse die befüllte Vertiefung der Luftkammer des Mundstückes zugewendet, fällt die Pulverdosis durch die Schwerkraft, unterstützt durch einen Rüttelmechanismus, aus der Vertiefung in einen Hohlraum des Luftkanals und wird von dort aus mit Hilfe aktiver Einatmung in die Lungen des Patienten inhaliert. Dabei weist der Luftkanal einen Drosselbereich auf, der das Mischen der Luft mit der medizinischen Substanz durch Turbulenz, d.h., das Bilden eines Aerosols, fördern soll.

Die Erzeugung des Aerosols erfolgt somit bei diesem Gerät durch aktives Einatmen in der Weise, daß die Einatmungsluft des Anwenders über das Pulver geleitet wird und dieses mitführt.

Das bekannte Inhalationsgerät weist zwei entscheidende Nachteile auf. Zum einen ist die zu inhalierende Dosis nicht ausreichend reproduzierbar. Zum anderen ist der Luftstrom, der durch die aktive Einatmung aufgebracht wird, trotz der unterstützenden Wirkung der Drosselstelle im Luftkanal nicht in der Lage, die Dosis vollständig aus dem Gerät herauszubringen und die medizinische Substanz im Luftstrom zu dispergieren. Dabei ist in Betracht zu ziehen, daß je nach Art des zu inhalierenden Pulvers dieses nur eine Partikelgröße von typischer Weise ca. 5 µm haben darf, um an den Wirkort in den Bronchien zu gelangen. Pulver mit einer solchen Kornfeinheit neigen jedoch beim Lagern zum Agglomerieren, so daß bei der Anwendung und nicht tiefgreifender Desagglomeration, wie dies beim bloßen Einatmen der Fall ist, mindestens ein Teil des Pulvers in Form von Agglomeraten mit einem größeren Durchmesser als er dem Primärkorn entspricht, inhaliert werden. Diese Agglomerate gelangen nicht an den Wirkort in den Bronchien, so daß ein beträchtlicher Dosierfehler entsteht, der bei der Anwendung hochwirksamer Pharmazeutika unzulässig ist.

Der Erfindung liegt die Aufgabe zugrunde, ausgehend von dem eingangs bezeichneten, treibgasfreien Inhalationsgerät dieses so zu gestalten, daß die bereitgestellte Dosis in hohem Maße reproduzierbar ist, ein hoher Anteil dieser Dosis das Inhalationsgerät verläßt und eine reproduzierbare Dispergierung der medizinischen Substanz erreicht wird.

Die Lösung dieser Aufgabe gelingt gemäß der Erfindung durch die Merkmale des Kennzeichnenden Teils des Anspruchs.

Das erfindungsgemäße Inhalationsgerät arbeitet mit einem dosierten, atemzugsynchron, selbstätig ausgelösten Fremdluftstrom. Der Druckluftstoß bläst die vorgegebene Dosis der zu inhalierenden medizinischen Substanz vollständig aus der Dosierkammer in den Luftkanal ein. Die Dosis ist daher bei den einzelnen Inhalationsvorgängen im hohen Maße gleich. Sie wird dabei durch den Druckluftstoß sehr fein verteilt, was die Inhalationswirkung beachtlich verstärkt. Sind Pulveragglomerate vorhanden, so werden diese aufgrund des Fremdluftimpulses desagglomeriert.

Zur Vermeidung des Nachteils nicht ausreichender Desagglomeration sind Geräte bekannt, in denen die Pulveragglomerate mittels eines Fremdluftimpulses desagglomeriert werden. Ein solches Gerät zeigt beispielsweise die WO-A-9007351. Zur Erzeugung dieses Fremdluftimpulses wird ein mittels Kolben oder Faltenbalg verdichtetes Luftvolumen sehr schnell freigesetzt. Der Luftimpuls reißt das Pulver mit, durch die dabei entstehenden Turbulenzen und Scherkräfte werden Agglomerate wieder zum Primärkorn desagglomeriert. Das desagglomerierte Produkt kann nun infolge seiner wieder erreichten Kornfeinheit tief in die Bronchien eindringen ohne nennenswerte Substanzverluste im Mund- und Rachenraum.

Bei der Aerosolerzeugung in der beschriebenen Weise ist es jedoch notwendig, den Fremdluftimpuls exakt im Augenblick des größten Atemluftstroms beim Einatmen auszulösen. Erfolgt die Auslösung nicht zu diesem Zeitpunkt, so resultiert daraus eine Fehlbedienung und Fehldosierung. Diese atemzugsynchrone Auslösung des Fremdluftimpulses wird durch die mechanische Schalteinrichtung erzielt, die auf Unterdruck beim Atmen anspricht.

Die Erzeugung eines Fremdluftstromes bei Inhalationsgeräten ist an sich aus der US-PS 3 921 637 bekanntgeworden. Bei dem bekannten Inhalationsgerät ist eine manuell betätigbare Balgenpumpe vorgesehen ist, die nur solange Luft mit erhöhtem Druck erzeugt, solange per Hand auf den Pumpenbalg Druck ausgeübt wird. Zwischen der Pumpe und einer Kammer, in der die zu inhalierende Dosis in Form von Kapseln eingebracht wird, ist bei dem bekannten Gerät eine Ventilanordnung vorgesehen, die von einer Schalteinrichtung betätigt wird, die auf den Atemluftstrom beim Einatmen anspricht. Im Gegensatz zur Erfindung betätigt daher diese Schalteinrichtung nicht den Auslösemechanismus einer vorgespannten Pumpe, sondern öffnet ein Ventil. Diese Schalteinrichtung besteht aus einer mittels Federn ausbalancierten Klappe, die über Hebel das Ventil öffnet.

Nach einer vorteilhaften Weiterbildung der Erfindung ist der Dosierkammer eine Dispergier-Düse nachgeschaltet, durch die der Fremdluftstrom geführt wird. Diese Düse sorgt für eine besonders gute Dispergierung und gegebenenfalls Desaggloerierung der medizinischen Substanz im Atemluftstrom.

In einer Ausgestaltung der Erfindung ist das Element für die manuelle Betätigung der Dosiereinrichtung mit der Aktivierung der Vorspanneinrichtung mechanisch gekoppelt. Durch diese Ausgestaltung ist das Gerät nach der Erfindung mit einer einzigen Spannbewegung hinsichtlich der Dosierung und der Atemlufttriggerung initialisierbar.

Eine weitere Ausgestaltung der Erfindung ist dadurch gekennzeichnet, daß die Pumpanordnung einen Pumpenraum mit einem Luftaustrittsstutzen und einem Pumpenkolben aufweist, welcher infolge der manuellen Betätigung gegen die Kraft einer Feder vorspannbar und auf der dem Luftaustrittsstutzen abgewendeten Seite des Pumpenraumes auslösbar verklinkbar ist. Eine derartige spannbare Kolbenpumpe ist eine besonders einfache und dennoch voll wirksame Ausbildung einer triggerbaren Pumpenanordnung für das erfindungsgemäße Gerät. Spannbare Kolbenpumpen sind an sich auch bei medizinischen Vorrichtungen bekannt (DE - 27 26 934 A1). Sie sind jedoch nicht selbsttätig und atemluftsynchron triggerbar.

Weitere ausgestaltende Merkmale sowie Vorteile der Erfindung ergeben sich anhand der Beschreibung von in den Zeichnungen dargestellten Ausführungsbeispielen.

Es zeigen:
- Fig. 1:: eine Ausführungsform des erfindungsgemäßen treibgasfreien Inhalationsgerätes im Normalzustand in einer geschnittenen Ansicht,
- Fig. 2:: in zwei verschiedenen Ansichten und in vergrößertem Maßstab die Ausbildung der in Figur 1 gezeigten Dosiskammer,
- Fig. 3:: eine Querschnittsansicht der Aufhängung der in Figur 1 gezeigten Düse im Luftkanal des Mundstückes,
- Fig. 4:: die Ausführungsform nach Figur 1 in der Bereitschaftsstellung zum Inhalieren,
- Fig. 5:: die Ausführungsform nach Figur 1 unmittelbar nach dem Einatmen der ausgeblasenen Dosis,
- Fig. 6:: in einer Explosionsdarstellung eine besondere Anordnung zur Dosierung des zu inhalierenden Wirkstoffes (Bandzug-Dosierung),
- Fig. 7:: die Dosieranordnung nach Figur 6 in eingebautem Zustand in einer Stellung, die eine Befüllung des Vorratsbehälters ermöglicht,
- Fig. 8:: die Dosieranordnung nach Figur 6 in einer Stellung, in der die Dosierkammer durch Drehen des Dosierstempels gefüllt wird,
- Fig. 9:: die Dosieranordnung nach Figur 6 in einer Stellung, bei der die in der Dosierkammer eingelagerte medizinische Substanz ausgetragen wird, und
- Fig. 10:: eine prinzipielle Darstellung einer alternativen Ausführungsform der Dosieranordnung nach Figur 6.
- Figuren 11 - 13:: ein weiteres Ausführungsbeispiel einer Bandzug-Dosierung in drei verschiedenen Ansichten
- Fig. 14:: ein weiteres Ausführungsbeispiel des erfindungsgemäßen treibgasfreien Inhalationsgerätes im Normalzustand in einer geschnittenen Prinzip-Ansicht.
- Fig. 15:: die Ausführungsform nach Figur 14 in der Bereitschaftsstellung zum Inhalieren.

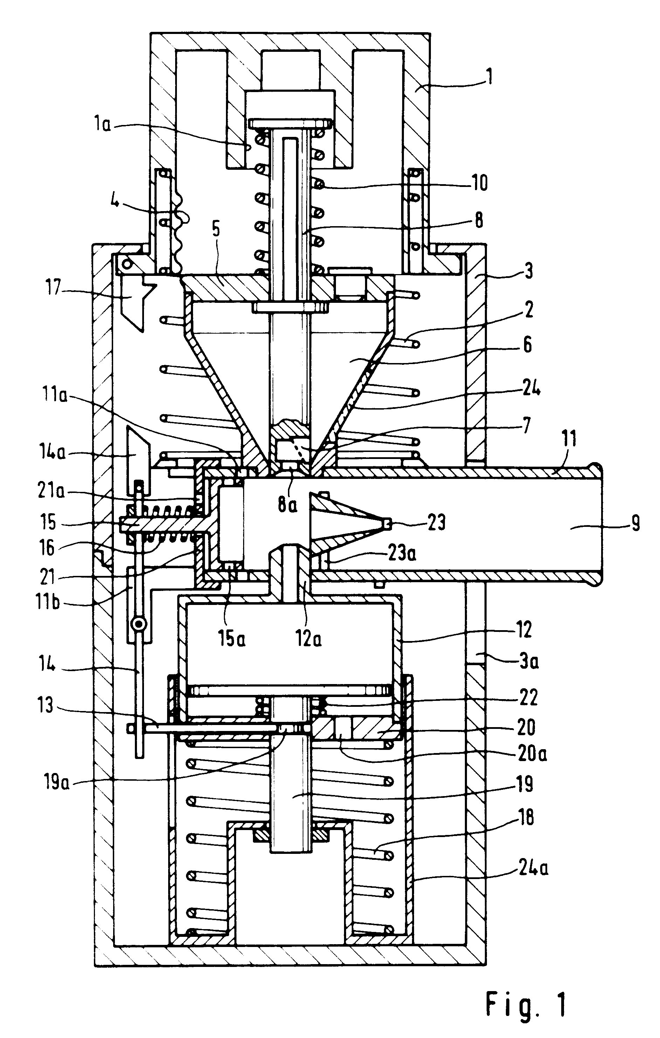

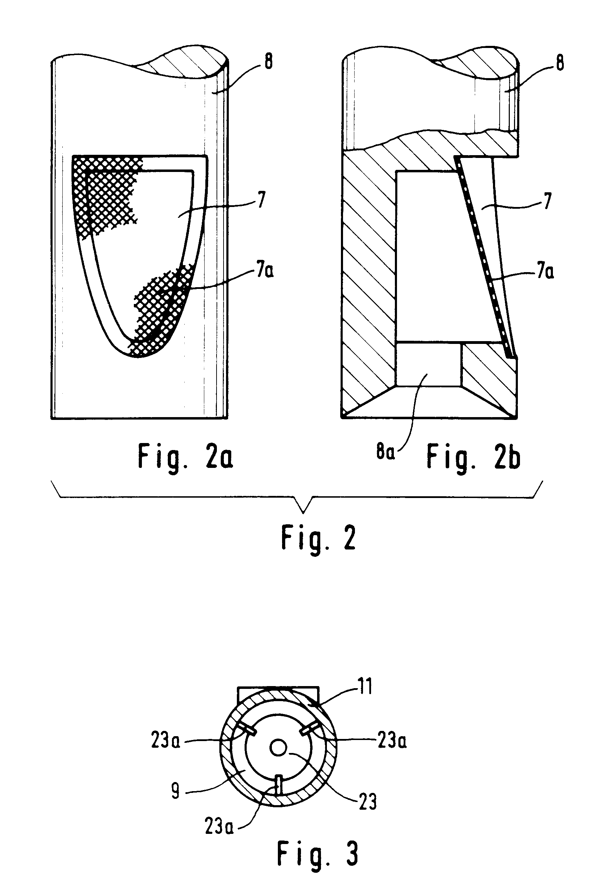

Das in Figur 1 dargestellte treibgasfreie Inhalationsgerät weist ein Gehäuse 3 mit einer seitlichen Öffnung 3a auf, in dem kopfseitig eine Drucktaste 1 gegen die Kraft einer Feder 2, die sich am Gehäuse abstützt, verschiebbar gehaltert ist. Die Drucktaste 1 weist an der Innenseite gezahnte oder gewellte Kanten 4 auf und besitzt ferner einen angeformten Hebel 17 zur Verklinkung mit einem Gegenstück am Gehäuse 3, an dem sich auch die Feder 2 abstützt. Das Inhalationsgerät besitzt ferner einen trichterförmigen Vorratsbehälter 6 als Vorratsraum für die zu inhalierende pulverförmige medizinische Substanz. Dieser Vorratsbehälter ist mittels eines Deckels 5 verschließbar und weist ein Fenster 24 zur Verbrauchsanzeige auf. Dem Vorratsbehälter ist ein Dosierstempel 8 zugeordnet, der eine Dosierkammer 7 zur Aufnahme der beim jeweiligen Inhalationsvorgang einzuatmenden Dosis an medizinischer Substanz aufweist. Diese Dosierkammer ist in den Figuren 2a und 2b in verschiedenen Ansichten näher dargestellt. Die Figur 2a - eine Ansicht parallel zu einer horizontalen Achse - läßt erkennen, daß die Dosierkammer im Querschnitt im wesentlichen dreieckförmig gestaltet ist. Sie weist, wie insbesondere die Querschnittsansicht nach Figur 2b erkennen läßt, eine Hinterschneidung auf, an der ein Siebboden 7a angebracht ist, der die Dosierkammer nach innen begrenzt.

Der Dosierstempel 8 durchdringt den Deckel 5 und das konusseitige Ende des Vorratsbehälters 6 und ist in ihm, gegen die Kraft einer Feder 10, die sich auf der anderen Seite in einer Aufnahme 1a in der Drucktaste 1 abstützt, verschiebbar gehaltert. Der Dosierstempel weist dosierkammerseitig eine Bohrung 8a für die Zufuhr von Luft zu der Dosierkammer 7 auf und ist an diesem Ende zum formschlüssigen Anschluß an einen Stutzen 12a eines Pumpengehäuses 12, welches später noch erläutert wird, entsprechend ausgeformt.

Der Deckel 5 der Vorratskammer hat eine abgerundete Kante, an der beim Herabdrücken der Drucktaste 1 die gewellte Kante 4 der Drucktasteninnenwand anstreifend vorbeigeführt wird, wodurch der Vorratsbehälter 6 gerüttelt wird. Auf diese Weise wird die gleichmäßige Befüllung der Dosierkammer 7 am Dosierstempel 8 erreicht.

Das Inhalationsgerät besitzt ferner ein seitlich angebrachtes Mundstück 11 zum aktiven Einatmen mit einem Luftkanal 9. In diesem Luftkanal ist eine Düse 23 über Stege 23a (siehe Figur 3) gehaltert. Das Mundstück 11 ist fest mit dem Vorratsbehälter 6 und dem Stutzen 12a des Pumpengehäuses verbunden und bildet mit diesem eine bewegliche Einheit. Es ist am linken Ende mit einem Deckel 21 verschlossen, der eine Entlüftungsbohrung 21a besitzt. Das Mundstück 11 weist an diesem deckelseitigen Ende ferner Belüftungsbohrungen 11a auf, deren Funktion noch erläutert wird.

Das Inhalationsgerät besitzt ferner eine Pumpenanordnung zur Erzeugung eines Druckluftstoßes (Fremdluftstromes) beim Inhalationsvorgang. Diese Pumpenanordnung weist das bereits erwähnte Pumpengehäuse 12 mit dem Stutzen 12a, aus dem die Luft ausgestoßen wird, auf. Das Pumpengehäuse ist durch einen Boden 20 mit einer Entlüftungsbohrung 20a abgeschlossen; in diesem Entlüftungsboden ist eine Entriegelungszunge 13 radialbeweglich gehaltert. Die Pumpenanordnung weist ferner einen Pumpenkolben 19 auf, der im Pumpenboden gegen die Kraft einer Feder 22 verschiebbar gehaltert ist. Der Pumpenkolben 19 besitzt eine umlaufende Nut 19a, in die die Entriegelungszunge 13 im gespannten Zustand der Feder 22 eingreift und den Pumpenkolben in gespanntem Zustand hält. Der Pumpenkolben 19 ist ferner in einem Gehäuseeinsatz 24a gehaltert, der eine Feder 18 aufnimmt, die sich am anderen Ende des Pumpengehäusebodens 20 abstützt.

Zum Auslösen der Pumpbewegung ist eine Triggermechanik vorgesehen, die in der dargestellten Ausführungsform selbsttätig beim aktiven Einatmen anspricht. Diese Triggermechanik weist einen Triggerkolben 15 auf, der gegen die Vorspannung einer relativ schwachen Feder 16 im Mundstück 11 gehaltert ist. Im Fortsatz des Triggerkolbens sind dabei Belüftungsbohrungen 15a vorgesehen. Ein weiteres Element der Triggermechanik ist der Entriegelungshebel 14, der an einem Fortsatz 11b des Mundstückes 11 drehbar gelagert ist. Dieser Entriegelungshebel 14 ist in der Kolbenstange des Triggerkolbens 15 beweglich geführt und besitzt kopfseitig ein Entriegelungselement 14a für den Drucktastenklinkenhebel 17. Er ist auf der anderen Seite in der Entriegelungszunge 13 beweglich geführt, die ebenfalls Teil der Triggermechanik ist.

Die Figur 1 zeigt das Inhalationsgerät im normalen Betriebszustand, d.h. im Zustand vor einem Gebrauch. Die Feder 22 des Pumpenkolbens 19 ist gespannt; die anderen Federn 2, 10, 16 und 18 befinden sich im entspannten Zustand.

Für die Einstellung des inhalationsbereiten Zustandes sind folgende Handhabungen vorzunehmen:
Die Drucktaste 1 wird manuell gegen die Kraft der Feder 2 heruntergedrückt. Bei diesem Vorgang wird die gezahnte oder gewellte Kante 4 am Behälterdeckel 5 anstreifend vorbeigeführt und damit der Pulver-Vorratsbehälter 6 gerüttelt und die Dosierkammer 7 befüllt. Beim weiteren Herabdrücken der Drucktaste 1 gegen die Kraft der Feder 10 wird der Dosierstempel 8 heruntergedrückt, bis dieser auf dem Pumpen-Stutzen 12a aufliegt. Hierbei wird die Feder 10 gespannt. Beim anschließenden vollständigen Herabdrücken der Drucktaste 1 wird die gesamte bewegliche Einheit, bestehend aus Pulver-Vorratsbehälter 6, Mundstück 11, Pumpengehäuse 12, Triggermechanik 13, 14, 15, 16 in die tiefste Position gebracht, wobei die Drucktaste mit ihrem angeformten Hebel 17 am Gehäuse arretiert wird. Beim vollständigen Herabdrücken wird die Feder 18 gespannt und der Pumpenkolben 19 wird samt der im Pumpengehäuseboden 20 befindlichen Entriegelungszunge 13 ebenfalls nach unten bewegt.

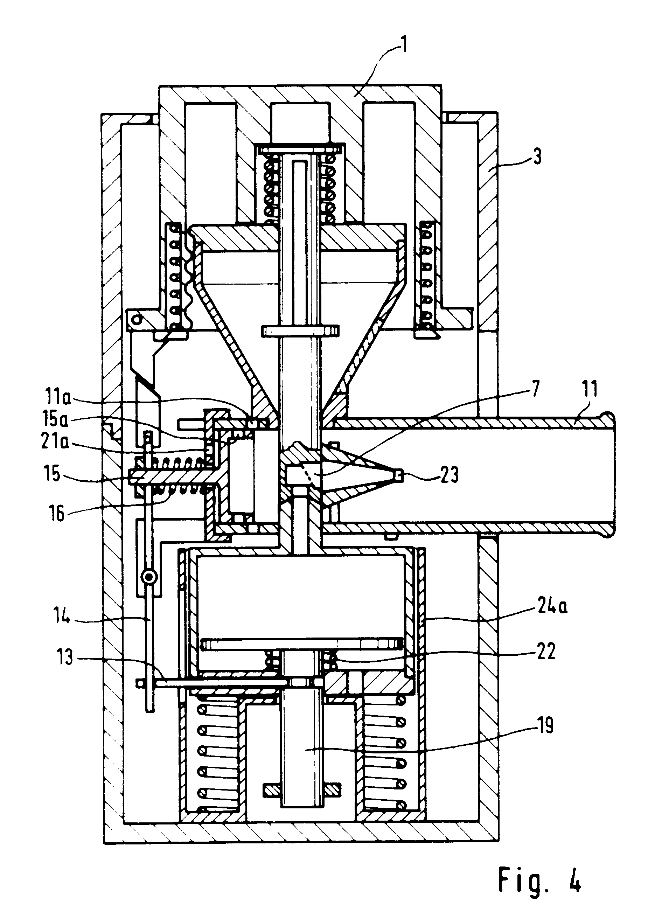

Das Inhalationsgerät befindet sich nun im Bereitschaftszustand, d.h. bereit zum Inhalieren. Dieser Betriebszustand ist in der Figur 4 dargestellt. Die Dosierkammer 7 befindet sich nunmehr unmittelbar auf Höhe der Düse 23. Sämtliche Federn, mit Ausnahme der dem Triggerkolben 15 zugeordneten Rückholfeder 16, sind gespannt.
Bei der Inhalation entsteht durch die aktive Einatmung vor dem Triggerkolben 15 im Mundstück 11 ein Unterdruck. Der Triggerkolben 15 wird gegen die schwache Feder 16 nach vorn bewegt und löst über den Entriegelungshebel 14 und die Entriegelungszunge 13 den Pumpenkolben 19 aus. Die Entlüftungsbohrung 21a im Mundstück-Deckel 21 verhindert dabei das Auftreten eines Unterdruckes an der Triggerkolben-Rückseite.

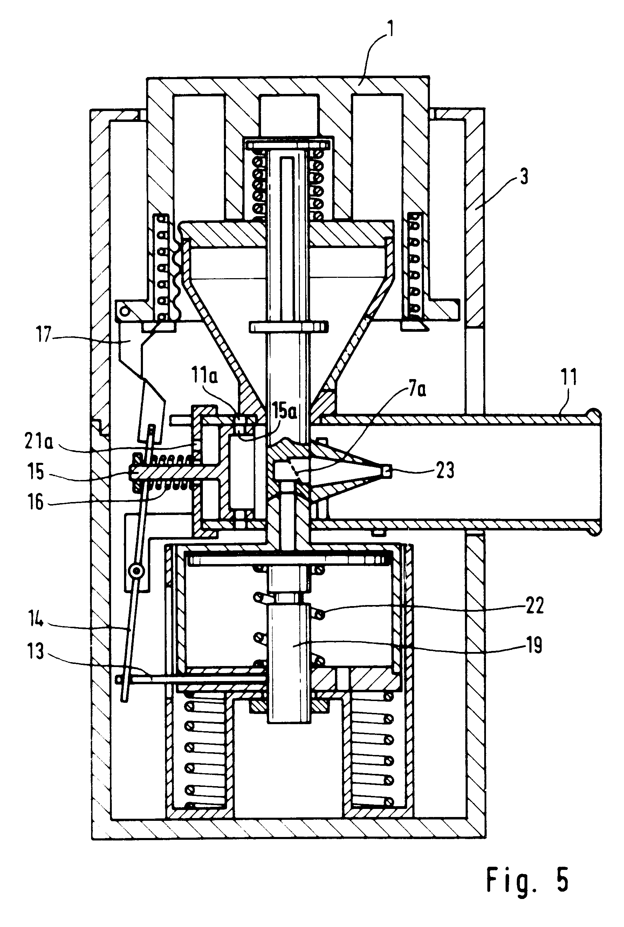

Der entriegelte Pumpenkolben 19 wird durch die Feder 22 schlagartig nach oben gedrückt. Das vom Pumpenkolben geförderte Luftvolumen wird hierbei stoßartig durch den Siebboden der Dosierkammer 7 gepreßt. Durch den Luftstoß wird das auf dem Sieb befindliche Pulver durch die Düse 23 ausgeblasen und dabei dispergiert. Beim weiteren aktiven Inhalieren kann Fremdluft über die Bohrungen 11a in der Mundstückwandung und 15a im Triggerkolben 15, die beim Ansaugen zur Deckung kommen, nachströmen, so daß das dispergierte Pulver mit dem Hauptatemluftstrom im Mundstück gemischt wird. Dieser Betriebszustand unmittelbar nach dem Gebrauch des Inhalationsgerätes ist in Figur 5 dargestellt.

Nach Beendigung des Inhalationsvorganges kehrt der Triggerkolben 15 selbsttätig durch Entspannung der Rückholfeder 16 in seine Ausgangsposition zurück. Dabei wird die Drucktaste 1 über den Klinkenhebel 17 durch den Hebel 14 entriegelt und kehrt aufgrund der Spannung der Federn 2, 10 sowie 18 und unter Spannen der Feder 22 in ihre Ausgangsposition nach Figur 1 zurück.

Die in den Figuren 1 bis 5 dargestellten Elemente des Inhalationsgerätes sind Ausführungsformen; die Erfindung ist darauf jedoch nicht beschränkt. Als mögliche Variante zu der in diesen Figuren dargestellten Triggermechanik kann der Triggerkolben 15 durch eine Klappe, der Hebelmechanismus 14 durch eine Kniehebelkonstruktion ersetzt werden.

In einer anderen Variante kann die Pumpeneinheit, bestehend aus den Teilen 12, 18, 19, 22 auch oberhalb des Mundstückes 11 angeordnet sein. Es ist desweiteren die Verwendung eines spannbaren Faltenbalges als Pumpeneinheit möglich.

Die Konstruktion des Inhalationsgerätes kann prinzipiell auch so getroffen werden, daß erst bei Betätigung der Drucktaste 1 der Pumpenkolben 19, d.h. die Feder 22 gespannt wird.

Die Ausführungsform nach Figur 1 ist auf eine selbsttätige Auslösung der Pumpe durch eine Triggermechanik beim Einatmen des Patienten abgestellt. Prinzipiell kann diese Triggermechanik auch durch eine manuell zu betätigende Auslösetaste ersetzt werden.

Eine Nachfüllung des Gerätes ist möglich. Hierzu kann die Baugruppe, bestehend aus dem Pulver-Vorratsbehälter 6, dem Dosierstempel 8 und der Feder 10, ausgetauscht werden. Hierbei ist entweder die Drucktaste 1 zu demontieren oder das Geräteoberteil abschraubbar zu gestalten. Alternativ hierzu kann der Deckel 5 des Pulver-Vorratsbehälters 6 so ausgebildet werden, z.B. mit einem Verschlußstopfen versehen werden, so daß Pulver aus einer Nachfüllpatrone nachgefüllt werden kann.

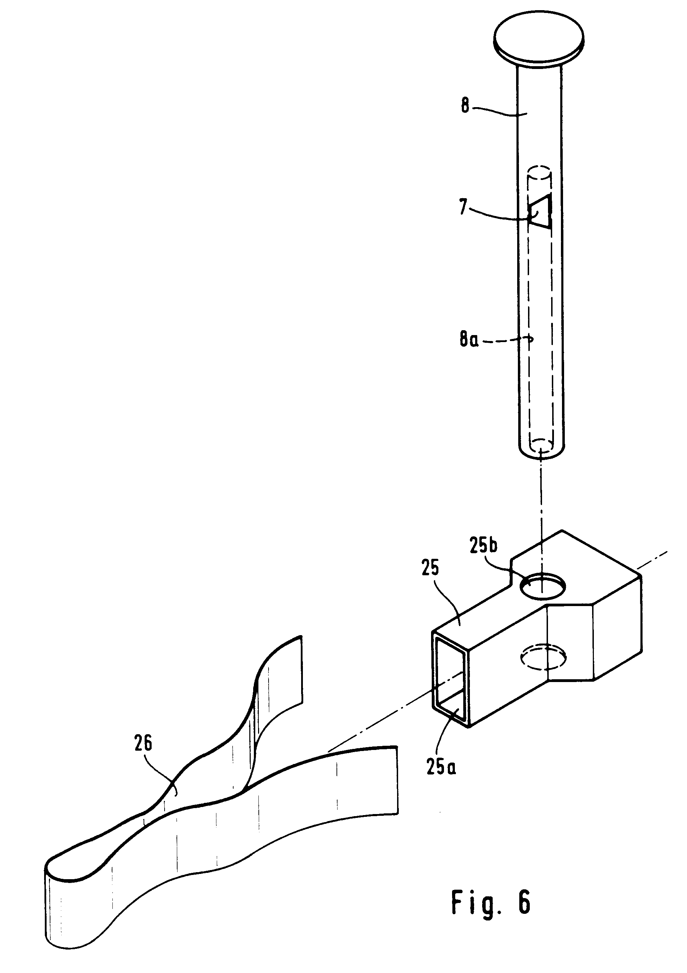

Auch die dargestellte Befüllung der Dosierkammer 7 am Ende eines trichterförmig ausgebildeten Vorratsbehälters ist eine von möglichen Varianten. Eine bevorzugte weitere Variante wird nunmehr anhand der Figuren 6 bis 10 näher beschrieben.

Die Dosierung schwer rieselfähiger Pulver (z.B. feinkörnige Glucose) durch Befüllen eines Dosiervolumens ist ohne aktive unterstützende Maßnahmen, wie z.B. das Rütteln des Vorratsbehälters nicht möglich. Die anschließend beschriebene - im folgenden "Bandzug-Dosierung" genannte - Dosiermethode ermöglicht für schwerrieselfähiges Pulver einen noch besseren Befüllungsgrad der Dosierkammer und damit eine bessere Reproduzierbarkeit der bereitgestellten Dosis.

Obwohl die Bandzug-Dosierung gerade bei dem erfindungsgemäßen Inhalationsgerät mit Vorteil Verwendung findet, kann sie grundsätzlich auch bei anderen Inhalationsgeräten angewendet werden.

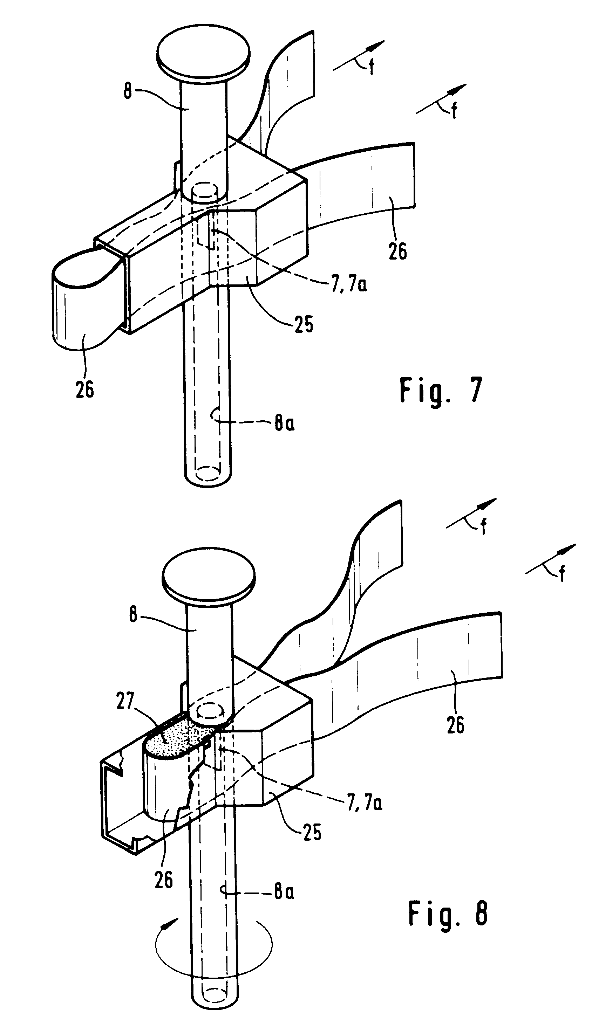

Die Figur 6 zeigt in einer Explosionsdarstellung die wesentlichen Elemente der Bandzug-Dosierung, nämlich ein Magazin 25 (das zum Beispiel den Vorratsbehälter 6 in Figur 1 ersetzt), ein Band 26, den Dosierstempel 8 mit der Dosierkammer 7 und eine Axialbohrung 8a, die vom Fußpunkt des Dosierstempels bis zur Dosierkammer 7 reicht. Der Dosierstempel ist, wie die Figur 7 erkennen läßt, in der Öffnung 25b des Magazins 25 aufgenommen. Das Band 26 wird an der schmalen Stirnseite des Magazins 25 mit den freien Enden eingeschlauft; diese durchlaufen das Magazin und treten an der gegenüberliegenden Stirnseite aus. Die in dem Magazin 25 eingelagerte Pulverschüttung 27 wird, wie die Figur 8 erkennen läßt, betrieblich von der Bandschlaufe rückseitig und seitlich umschlossen und erfährt entlang der Magazinlängsachse einen Vorschub "f" in Richtung Dosierstempel 8 bzw. Dosierkammer 7. Das Band besteht aus einem hinreichend zug- und reißfesten, vorzugsweise beidseitig silikonisierten oder teflonisierten Papier mit definierter Oberflächenrauhigkeit. Dieses soll die Haftkräfte des Pulvers auf dem Band herabsetzen, sowie die Reibung zwischen der Wandung des Magazins, dem Papier und dem Dosierstempel minimieren.

Das Magazin kann rückseitig in einer Stellung des Bandes entsprechend Figur 7 befüllt werden; die Pulverschüttung wird anschließend durch Anziehen der Bandschlaufe leicht gegen den Dosierstempel verdichtet.

Die Dosierung erfolgt mittels einer speziell geformten Dosierkerbe 7 als Dosierkammer in dem Dosierstempel 8, welche an der leicht verdichteten Pulverschüttung 27 (Figur 8) in einer gemäß der Kerbengeometrie vorgegebenen Vorzugsrichtung (im Uhrzeigersinn) vorbeigedreht und gefüllt wird. Eine weitere Ausführungsform besteht darin, daß der Dosierstempel 8 eine Hubbewegung ausführt. In dieser Ausführungsform ist die Dosierkerbe vorzugsweise 45° gegen die Dosierstempellängsachse geneigt. Die Dosierkerbe ist dabei rückseitig zur axialen Bohrung 8a hin z.B. mit einem feinen Edelstahl-Siebgewebe 7a - ähnlich der Anordnung nach Figur 2b - hinterlegt. Die Maschenweite beträgt vorzugsweise ca. 5 - 300 µm, vorzugsweise ca. 50 µm.

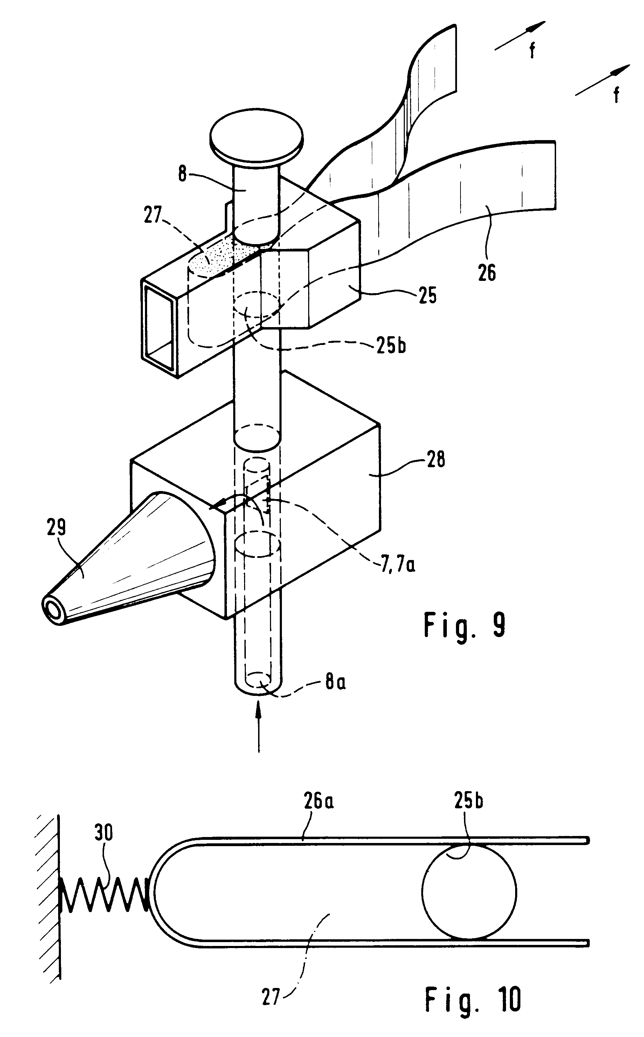

Für das Austragen der Dosis aus der Dosierkerbe 7 gibt es mehrere Möglichkeiten. Eine davon, die an das Ausführungsbeispiel der Figur 1 angelehnt ist, zeigt die Figur 9. Nach Befüllung der Dosierkerbe 7 durch die beschriebene Dreh- oder Hubbewegung wird der Dosierstempel 8 durch die Bohrungen 25b des Magazins 25 - analog dem Übergang vom Betriebszustand nach Figur 1 zum demjenigen nach Figur 4 - soweit heruntergedrückt, daß die Dosierkerbe 7 in einem kleinen, speziell geformten, hier nicht näher ausgeführten Dispergiervolumen 28 vor einer ebenfalls nicht näher beschriebenen, speziell geformten Düse 29 positioniert wird. Der Pulverabstreifvorgang beim Durchdrücken des Dosierstempels durch die Bohrungen 25b des Magazins bewirkt eine weitere Präzisierung der Dosis.

In der Stellung nach Figur 9 wird über die Bohrung 8a ein Druckluftstoß auf den Dosierkerbenboden, das Siebgewebe 7a, ausgeübt, um das in der Kerbe auf dem Siebgewebe eingelagerte Pulver auszublasen. Der Druckluftstoß kann z.B. durch eine Pumpenanordnung nach Figur 1 erzeugt werden; auch andere Ausführungsformen sind denkbar.

Die freien Volumina der Magazin-Vor-(schmale Seite)- und Rückseite (breite Seite) können zum Feuchteschutz des eingelagerten Pulvers mit Trockenmittel befüllt werden.

Zur genaueren Dosierung des Wirkstoffes kann dieser auch mit einem Trägerstoff vermischt werden.

Wesentliches Merkmal der Bandzug-Dosierung ist, daß sich die Wand des langsam entleerenden Magazins beim Befüllen der Dosierkerbe gemeinsam mit der Pulverfüllung in Förderrichtung mitbewegt. Das Band 26 kann grundsätzlich auch durch eine starre Wand, z.B. eine U-förmige Klammer 26a, ersetzt werden (Figur 10), die unter dem leichten Druck einer Feder 30 steht und dem vorrätigen Volumen nachfolgt.

Wird die Bandzugdosierung im Rahmen einer Ausführung nach Figur 1 verwendet, ist die lineare Bewegung der Drucktaste 1 hinsichtlich des Dosierstempels 8 in eine Drehbewegung umzusetzen.

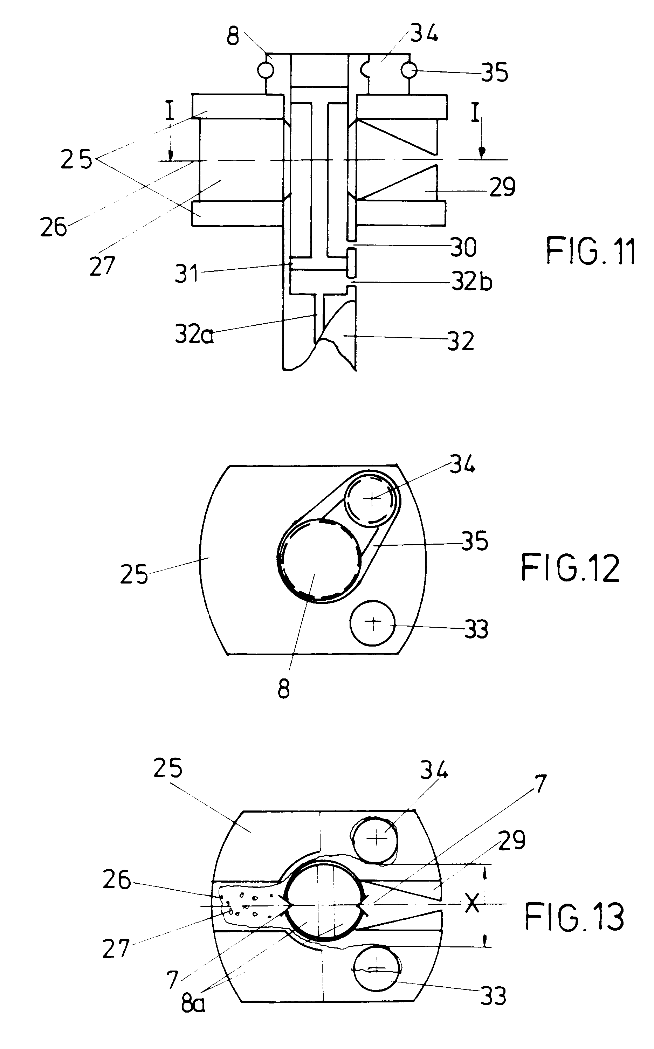

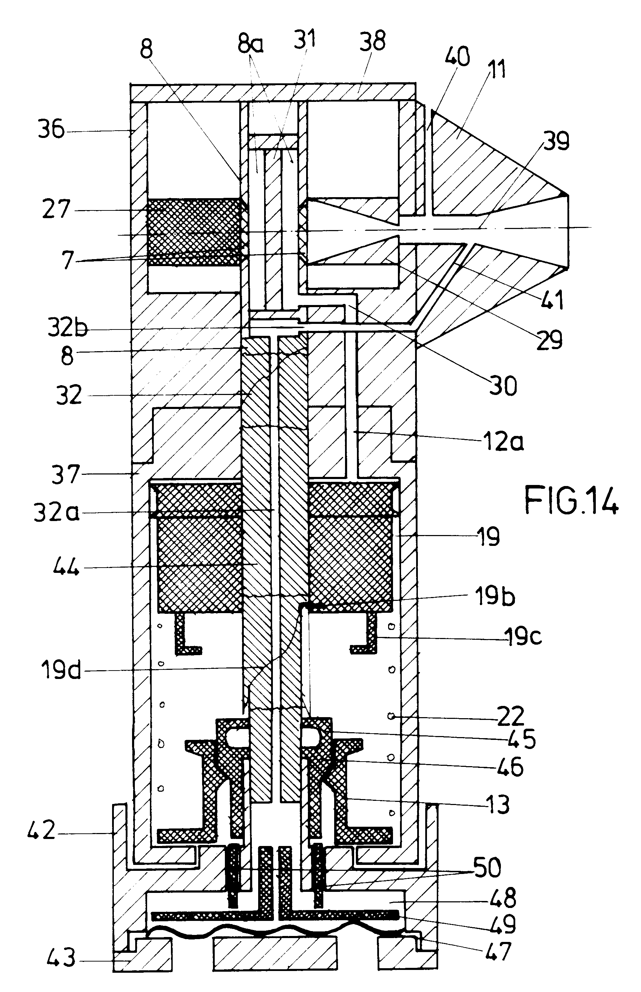

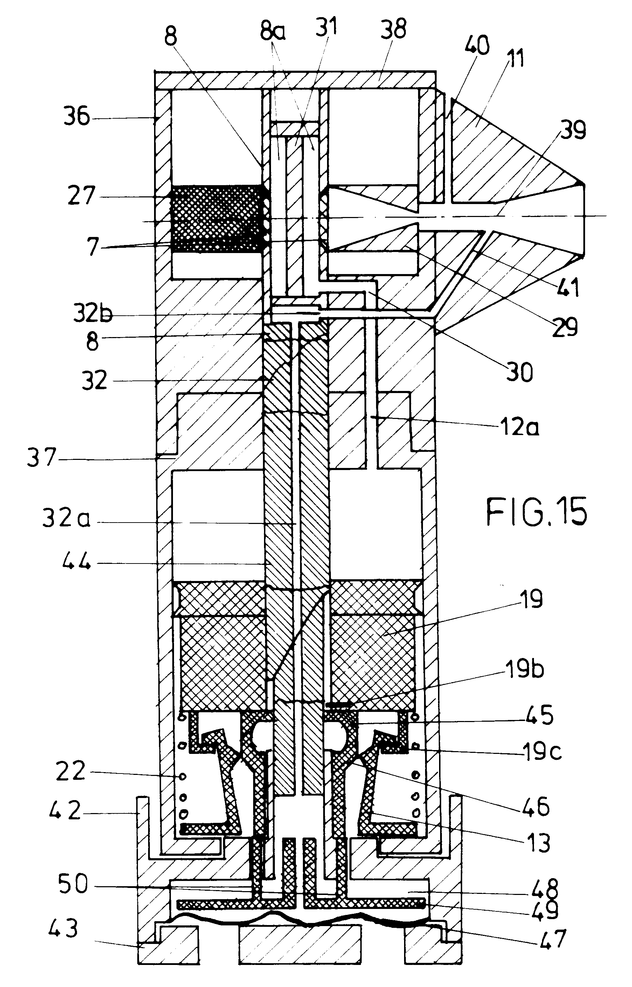

In den Figuren 11 - 13 ist ein weiteres, das bevorzugte Ausführungsbeispiel der Bandzugdosierung, dargestellt. Dieses Ausführungsbeispiel ist gegenüber den Prinzipdarstellungen in den Fig. 7 - 9 konstruktiv noch verfeinert. Es ist integraler Bestandteil des in den nachfolgenden Figuren 14 und 15 dargestellten weiteren Ausführungsbeispieles des erfindungsgemäßen Inhalationsgerätes welches - gegenüber den Ausführungen in den Figuren 1 bis 5 - das bevorzugte Ausführungsbeispiel ist.

Um die prinzipiellen Gemeinsamkeiten der Ausführungsbeispiele deutlich hervortreten zu lassen, wurden funktionsgleiche Elemente mit derselben Bezugsziffer versehen, auch wenn sie konstruktiv nicht völlig übereinstimmen.

Die Figuren 11 bis 13 zeigen ein doppelscheibenförmiges Gehäuse des Magazins 25 der Bandzugdosierung, wobei der Abstand zwischen den beiden Scheiben der Breite des Bandes 26 entspricht.

Die Figur 11 zeigt dieses Magazin in einem Längsschnitt, die Figur 12 stellt eine Draufsicht auf das Magazin nach Figur 11 dar und Figur 13 zeigt einen Schnitt entlang der Linie I in Figur 11.

Das im Magazin eingeschlaufte Band 26 umfaßt den Pulvervorrat, die Pulverschüttung 27 (Figur 13), die auf der rechten Seite durch den Dosierstempel, ein Dosierstift 8, begrenzt wird. Der Dosierstift 8 durchdringt drehbar das Magazin 25 und weist in diesem Ausführungsbeispiel zwei Dosierkerben 7 auf. Es sind auch Ausführungen mit mehr als zwei über den Dosierumfang verteilte Dosierkerben möglich. Die Dosierkerben sind dabei zweckmäßig rückseitig mit einem Sinterkunststoff, -glas, -metall, einem Netz oder einem Sieb verschlossen. Der Dosierstift 8 weist ferner eine der Zahl der Dosierkerben entsprechende Anzahl von Kammern 8a auf, derart, daß stets nur die "auszublasende" Dosierkerbe über die zugeordnete Kammer anströmbar ist, insbesondere mit dem dispergierenden Druckluftstoß aus einer triggerbaren Pumpe. Die beiden Kammern, getrennt durch Wandungen 31, sind in Figur 11 schematisch dargestellt, wobei die Öffnung 30 den Einlaß für die Dispergierluft darstellt. Im unteren Teil des Dosierstiftes 8 befindet sich ein Kupplungsteil 32, über den der Dosierstift mit einem Drehantrieb verbindbar ist. Dieser Abschnitt des Dosierstiftes weist eine Bohrung 32b für einen Unterdruckkanal und eine Längsbohrung 32a zur Weiterleitung des Unterdruckes auf, deren Bedeutung im Zusammenhang mit den Figuren 14 und 15 noch erläutert wird.

Mit der auszublasenden Dosierkerbe 7 bzw. der zugeordneten Kammer im Dosierstift 8 steht die Dispergierdüse 29 in Verbindung. Die Anbringung der Düse im Magazin ist zweckmäßig durch eine geeignete (nicht dargestellte) Vorrichtung, z.B. eine Nocke, so getroffen, daß die Düse beim Bandtransport und damit beim Drehen des Dosierstiftes 8 im Bereich des Eintretens der Dosierkerbe 7 in den Düsenbereich, leicht vom Dosierstift abhebt, um Pulverabstreifungen zu vermeiden.

Von besonderer Bedeutung für die Funktionsfähigkeit der Bandzugdosierung ist der Bandvorschub. Das Ausführungsbeispiel nach den Figuren 11 - 13 sieht eine entsprechend günstige konstruktive Lösung vor. Sie zeigt einen Stift 33, an dem das eine Ende des Bandes befestigt ist. Das andere Ende des Bandes ist am Bandspannstift 34 aufwickelbar befestigt. Der Bandspannstift 34, die Aufwickelachse, ist mit dem Dosierstift 8 über einen Riemen 35 gekoppelt. Anstelle des Riemenantriebes kann auch ein Zahnradantrieb oder dergleichen verwendet werden. Durch Drehen des Dosierstiftes 8 wird der Bandspannstift 34 durch den Riemen 35 angetrieben. Dabei spannt sich das Band 26 und verdichtet den Pulvervorrat 27, bis ein bestimmter Bandzug auftritt. Beim Erreichen dieses Bandzuges rutscht der Riemen nach Art einer Rutschkupplung auf dem Bandspannstift 34 durch. Während dieses Vorganges wird die Dosierkerbe 7 befüllt.

Wesentlich ist dabei, daß die Kraft, mit der die Bandschlaufe die leicht verdichtete Pulverschüttung 27 gegen den Dosierstift 8 preßt, limitiert ist und daß das Pulver nur zum Zeitpunkt der Dosierung mit Druck beaufschlagt wird. Dadurch wird ein "Verkeilen" des Pulvervorrates 27 bzw. des zugehörigen Bandteiles der Schlaufe im Magazin 25 verhindert.

Ein besonderes Konstruktionsmerkmal ist auch die Größe der Bandumschlingung am Dosierstift 8. Die Umschlingung des Dosierstiftes ist zweckmäßig derart gewählt, daß sie über den (senkrechten) Durchmesser des Dosierstiftes (Figur 13) hinausgeht, d.h., daß der Umschlingungswinkel größer als 180° ist. Dies wird dadurch erreicht, daß das Maß X, der innere freie Abstand zwischen den Stiften 33 und 34, kleiner als der Außendurchmesser des Dosierstiftes 8 ist. Mit dieser Maßnahme wird eine Verschmutzung des Stempels weitgehend verhindert und ein sauberes Befüllungsbild der Dosierkerbe erreicht.

Ein bedeutsamer Vorteil der Bandzugdosierung ist es, daß sie lageunabhängig ist.

Die Figuren 14 und 15 zeigen ein weiteres Ausführungsbeispiel des erfindungsgemäßen Inhalationsgerätes, bei dem eine Bandzugdosierung nach den Figuren 11 bis 13 vorgesehen ist.

Dieses Inhalationsgerät weist ein zweiteiliges Gehäuse auf, nämlich das Kopfteilgehäuse 36, das oben mit einem Deckel 38 abgeschlossen ist, und das Pumpengehäuse 37, das gleichzeitig den Zylinder für den Kolben 19 der Pumpanordnung bildet. Beide Gehäuseteile bestehen vorzugsweise aus Kunststoff und weisen übliche nicht dargestellt, Verbindungselemente, z.B. eine Schraubverbindung, auf.

Im Kopfteilgehäuse 36 ist die Bandzugdosierung gemäß den Figuren 11 bis 13 untergebracht, die an dieser Stelle daher nicht weiter erläutert werden muß. Am Kopfteilgehäuse ist ferner das Mundstück 11 angebracht, das der Dispergierdüse 29 der Bandzugdosierung gegenüber liegt. Das Mundstück 11 besitzt einen Luftkanal in Form einer Inhalationsdüse 39 sowie Atemluftbohrungen 40 für die nachströmende Außenluft beim Inhalieren. Das Mundstück 11 weist ferner einen Unterdruckkanal 41 auf, der mit der Bohrung 32b am Dosierstift 8, (Fig. 11) und damit mit der Längsbohrung 32a in Verbindung steht. Beim aktiven Einatmen entsteht infolge der Beschleunigung, die die Luft in der Düse erfährt, in der Düse und damit im Kanal 41 ein Unterdruck, der sich in die Längsbohrung 32a fortsetzt. Der Unterdruckkanal befindet sich daher zweckmäßig an der Stelle in der Düse, in der die höchste Geschwindigkeit herrscht.

Die zweigeteilte Ausführung des Inhalationsgerätes erlaubt ein schnelles Auswechseln des Kopfteilgehäuses, wenn der Vorrat an medizinischer Substanz aufgebraucht ist oder ein Austausch gegen andere Kopfteilgehäuse, die mit unterschiedlichen medizinischen Substanzen in der jeweiligen Bandzugdosierung befüllt sind.

Das Pumpengehäuse 37 weist einen Drehknopf 42 auf, der mit einer Spannwelle 44 verbunden und stirnseitig durch einen mit Bohrungen versehenen Deckel 43 abgeschlossen ist. Die Spannwelle 44 weist, wie der Dosierestift 8, mit dem sie über die Kupplung 32 positionsgenau und torsionskraftschlüssig verbindbar ist, die Axialbohrung 32a auf.

In dem Pumpengehäuse 37 ist im vorderen Teil die spann- und triggerbare Kolbenpumpe untergebracht. Die Pumpe weist den Pumpenkolben 19 auf, der einen Stift 19b besitzt, der in einer Spiralnut 19d der Spannwelle 44 geführt ist. In kinematischer Umkehr dieses Prinzips der translatorischen Umsetzung einer Drehbewegung können an der Spannwelle Nocken oder dergleichen vorgesehen sein, die in einer Spiralnut aufgenommen sind, die sich in der Kolbenbohrung befindet.

In Figur 14 befindet sich der Kolben in der oberen Stellung nach der Triggerung und dem Luftausstoß, wogegen die Figur 15 den gespannten Kolben zeigt. In dieser Darstellung ist besonders gut der Pumpzylinderraum oberhalb des Kolbens 19, in dem die Luft durch den hochgehenden Kolben komprimiert wird, zu erkennen. An dem Pumpzylinderraum befindet sich die Luftaustrittsöffnung, der Druckkanal 12 a, der in die Bohrung 30 am Dosierstift 8 zwecks Weiterleitung der dispergierenden Druckluft in die jeweilige Kammer 8a bzw. Dosierkerbe 7 einmündet (siehe auch Figur 11).

An der unteren Kolbenseite ist ein radialsymetrisches Klinkelement 19c, eine Halteklammer vorgesehen, mit dem der Kolben gegen die Kraft der Feder 22 unter Eingriff mit einer ebenfalls radialsymetrischen Verriegelungsklammer 13, die elastisch nachgebende Segmente besitzt, vorspannbar ist.

Die aufeinanderliegenden Teile der Halte- und Verriegelungsklammer weisen eine leichte Schräge auf, derart, daß die Halteklammer 19c unter dem Einfluß der Kraft der Feder 22 das Bestreben hat, die Segmente der Verriegelungsklammer nach innen zu bringen und damit die Verriegelung zu öffnen. Die Schräge unterstützt daher die Auslösung, zusätzlich unterstützt durch die Eigenspannung der Segmente der Verriegelungsklammer. Der Verklink- und Auslösemechanismus weist ferner einen Rückstellknopf 45, der sich mit der Spannwelle 44 mitdreht, sowie einen Auslöseknopf 46 mit einer Verriegelungsschulter, der längsaxial verschiebbar ist, auf. Beim Spannen des Kolbens wird der Auslöseknopf 46 über den Rückstellknopf 45 oberhalb seiner Verriegelungsschulter in die Verriegelungs- und zugleich Auslöseklammer 13 gedrückt, derart, daß die Auslösekante der Auslöseklammer 13 oberhalb der Verriegelungsschulter des Auslöseknopfes 46 liegt. Dabei rastet die Verriegelungs- und Auslöseklammer in das Klinkelement oder Haltklammer 19c des Kolbens ein (Figur 15).

Die aufeinandergleitenden, mit einer Kulisse versehenen Kanten von Rückstell- und Auslöseknopf 45 und 46 sind rampenartig gestaltet. Im gespannten Zustand (Figur 15) ist der höchste Punkt der Rampe bereits überschritten, so daß der Raum hinter dem Rampenabbruch für die notwendige axiale Bewegung des Auslöseknopfes beim Auslösen zur Verfügung steht.

Die dargestellte Verklinkung stellt eine verhältnismäßig einfache Lösung dar, die auch maschinell leicht montierbar ist.

Zum selbsttätigen Auslösen der Pumpe beim aktiven Einatmen ist eine besonders vorteilhafte Triggermechanik vorgesehen, die als zentrales Element eine Membrane 47 aufweist, die auf den beim Einatmen entstehenden Unterdruck im Unterdruckkanal 41 bzw. fortpflanzend in der Axialbohrung 32a, anspricht. Die Membrane 47 begrenzt stirnseitig einen Membranraum 48, in dem sich ein Membrantopf 49 befindet, an dem Auslösestifte 50 anliegen, die im Drehknopf 42 geführt sind. Mit ihrem anderen Ende liegen diese Auslösestifte am Auslöseknopf 46 an. Diese Membrantriggerung kann grundsätzlich auch bei anderen Inhalationsgeräten bzw. zur mechanischen Triggerung anderer technischer Vorgänge verwendet werden.

Für die Einstellung des inhalationsbereiten, gespannten Zustandes gemäß Figur 15 aus dem ungespannten Ruhezustand nach Figur 14 sind folgende Handlungen vorzunehmen:
Der Drehknopf 42 wird manuell um einen bestimmten Winkel verdreht. Mit dem Drehknopf drehen sich mit die Spannwelle 44, der Rückstellknopf 45 und der Dosierstift 8 (über die Kupplung 32). Im Ausführungsbeispiel ist der Steigungswinkel der Spiral-Nut 19d so gewählt, daß eine Verdrehung von 180° notwendig ist, um den Spannzustand zu erreichen.

Durch die Drehung des Drehknopfes 42 wird zunächst über die Drehung des Dosierstiftes 8 in der Bandzugdosierung im Kopfteilgehäuse 36 die Dosierkerbe 7 mit dem zu inhalierenden Pulver befüllt, wie es bereits anhand der Figuren 11 bis 13 im einzelnen beschrieben ist. Weiterhin wird durch das Drehen der Spannwelle 44 der in der Spiralnut 19d der Spannwelle geführte Stift 19b des Kolbens 19 nach unten bewegt. Dabei spannt der Kolben 19 die Feder 22. Nach einem Drehwinkel von ca. 135° befindet sich der Kolben an sich in der Ausgangsstellung. Beim Drehen des Knopfes 42 um 45° wird der Auslöseknopf 46 über den Rückstellknopf 45 mit seiner Verriegelungsschulter in die Auslöseklammer 13 gedrückt. Dabei rastet diese Klammer in das Klinkelement 19c des Kolbens kraftschlüssig ein.

Der Kolben ist jetzt gespannt und wird durch die beschriebene Verriegelung in seiner Position gehalten. Die Schrägen an der Verriegelungsklammer 13 und an dem Klinkelement 19c sind so ausgeführt, daß das Klinkelement unter dem Einfluß der Federkraft das Bestreben hat, die Verriegelungsklammer nach innen zu bringen und damit die Verriegelung zu öffnen. Dies wird jedoch durch den Auslöseknopf 46 verhindert, dessen dickeres Oberteil gegen die Nocken der Verriegelungsklammer drückt und diese im gespreizten Zustand hält. Dadurch ergibt sich eine besonders vorteilhafte Sicherheit gegen unbeabsichtigtes Fehlauslösen des Gerätes.

Das Inhalationsgerät befindet sich nun im Bereitschaftszustand nach Figur 15, d.h. ist bereit zum Inhalieren.

Bei der Inhalation wird beim Einatmen durch das Mundstück 11 über die Fremdluftöffnung 40 Luft zugeführt. Durch diese an der Bohrung 41 und der Düse 39 vorbeiströmende Umgebungsluft entsteht ein Unterdruck in dieser Bohrung 41, der über die Axialbohrung 32a bis in den Membranraum 48 weitergeführt wird. Durch den atmosphärischen Druck, der über die Bohrungen im Deckel 43 anliegt, wird die Membrane 47 nach innen auf den Membrantopf 49 gedrückt. Dieser drückt auf die Auslösestifte 50, die ihrerseits am Auslöseknopf 46 anliegen und bei Erreichen eines bestimmten Unterdruckes auslösen, indem die Verriegelungsschulter des Auslöseknopfes 46 durch eine Axialbewegung dieses Knopfes über die Auslösekante (Nocke) der Verriegelungsklammer 13 gebracht wird. Die Nocken der Verriegelungsklammer 13 gelangen dabei in den Bereich des dünneren Schaftes des Auslöseknopfes und können sich nicht mehr an diesem abstützen. Unter dem Einfluß der Eigenspannung der Segmente der Verriegelungsklammer, die bestrebt ist die Federsegmente nach innen zu biegen, und unter dem Einfluß der an den Schrägen der Verriegelungsklammer und des Klinkelementes 19c auftretenden, nach innen gerichteten Kraft biegen sich die Segmente der Verriegelungsklammer nach innen und der Kraftschluß zwischen der Klammer und dem Klinkelement 19c wird aufgehoben.

Durch diese doppelt zur Entriegelung wirkenden Kräfte wird vorteilhafterweise eine besonders hohe Auslösesicherheit bewirkt.

Der Kolben 19 wird durch die Kraft der Feder 22 nach oben bewegt. Der entstehende Luftdruckstoß wird durch den Druckkanal 12a weitergeleitet und gelangt über die Bohrung 30 in die rechte Kammer 8a in der Banddosierung. Das in der rechten Dosierkerbe 7 befindliche Pulver wird über die Düse 29 dispergiert und dem Atemluftstrom beigemischt, d.h. in ein Aerosol überführt. Danach befindet sich das Gerät wieder im Ausgangszustand nach Figur 14.

Die in den Figuren 14 und 15 verwendeten Baugruppen und -elemente sind Ausführungsformen; die Erfindung ist darauf jedoch nicht beschränkt. So können beispielsweise auch andere Konstruktionselemente zum Umsetzen einer Drehbewegung in eine Längsverschiebung des Kolbens 19 bzw. andere Verklink- und Auslösemechanismen verwendet werden, ohne daß die Erfindung deswegen verlassen würde.

Anhand der Figur 14 und 15 wird ein weiterer Vorteil des erfindungsgemäßen Gerätes deutlich. Das eingangs beschriebene bekannte Inhalationsgerät mit atemzugssynchroner Auslösung weist eine Vielzahl sehr präzise gearbeiteter Teile auf, die in einem aufwendigen Montageverfahren zusammengefügt und justiert werden müssen. Im Interesse einer preiswerten Herstellung solcher Geräte ist jedoch eine sehr einfache Geräteausführung anzustreben. Insbesondere ist es notwendig solche, in großer Stückzahl herzustellenden Geräte maschinell in einem schnellen Arbeitstakt zu montieren.

Als gravierenden Nachteil ist bei dem bekannten Gerät (US-PS 3921637) ferner zu werten, daß die Atemluft durch das Gerät und über die mechanischen Teile strömt. Dadurch ist im Laufe der Zeit eine Staub- und Schmutzablagerung auf den präzisen Lagerstellen unvermeidbar. Dies kann insbesondere auch dadurch auftreten, daß Geräte dieser Art in den Kleidungstaschen mitgeführt werden. Jede Verschmutzung der empfindlichen, mechanischen Teile kann jedoch die Funktionsfähigkeit solcher Geräte in Frage stellen, was z.B. etwa bei einem Asthmaanfall dramatische Folgen haben kann.

Geräte der gattungsgemäßen Art dürfen bei der Inhalation der Atemluft nur einen geringen Widerstand entgegensetzen. Daraus resultiert, daß für die Auslösung des Fremdluftimpulses nur geringe Kräfte zur Verfügung stehen. Von allen mechanischen Teilen ist somit eine außerordentliche Leichtgängigkeit zu fordern.

Die Nachteile bekannter Inhalationsgeräte werden bei dem erfindungsgemäßen Gerät vermieden bzw. es werden die angestrebten Ziele erreicht. Die Atemluft bzw. die Fremdluft hat einen sehr kurzen Weg zum Mundstück. Die Fremdluft durchströmt vorher nur den Dosierstift und die Dosierkammer. Eine Staub- und Schmutzablagerung wird daher vermieden, die Teile bleiben leichtgängig. Bei einer dringend erforderlichen Applikation des Aerosols, etwa bei einem akuten Asthmaanfall, ist somit eine außerordentlich einfache und schnelle Handhabung des Gerätes möglich.

Die Teile der Konstruktion sind ferner verhältnismäßig einfach und auch leicht zu montieren, damit ist das Gerät kostengünstig in großer Stückzahl herstellbar.

## Patentansprüche

1. Treibgasfreies Inhalationsgerät, mit einem Vorratsraum (6) für eine zu inhalierende, pulverförmige medizinische Substanz, ihm zugeordnet eine manuell betätigbare Dosiereinrichtung (8) zur Aufnahme einer vorgegebenen Dosis der medizinischen Substanz für den jeweiligen Inhalationsvorgang in mindestens einer Dosierkammer (7), und mit einem seitlichen Mundstück (11) zum aktiven Einatmen, welches einen Luftkanal (9) zur Verteilung der jeweiligen Dosis der medizinischen Substanz im Luftstrom aufweist, dadurch gekennzeichnet, daß eine triggerbare Pumpanordnung (12, 12a, 19, 20, 22) in Zuordnung zur Dosierkammer (7) vorgesehen ist, mit einer manuell aktivierbaren Vorspanneinrichtung (13, 19a; 19c) und einer mechanischen Schalteinrichtung (14 - 16; 47 - 50), die mit der Vorspanneinrichtung und dem Luftkanal (9) im Mundstück (11) in Wirkverbindung steht, derart, daß die Schalteinrichtung auf den beim Einatmen erzeugten Unterdruck anspricht und die Vorspanneinrichtung unter Erzeugung eines die befüllte Dosierkammer (7) ausblasenden, die Substanz dispergierenden, Fremdluftstromes auslöst.

2. Inhalationsgerät nach Anspruch 1, dadurch gekennzeichnet, daß der Dosierkammer (7) eine Dispergierdüse (23; 29) nachgeschaltet ist, durch die der Fremdluftstrom geführt wird.

3. Inhalationsgerät nach Anspruch 1 oder 2 mit einem mechanischen Element (1, 42) für die manuelle Betätigung, dadurch gekennzeichnet, daß das Element (1, 42) für die manuelle Betätigung der Dosiereinrichtung (8) mit der Aktivierung der Vorspanneinrichtung (13, 19a; 19c) mechanisch gekoppelt ist.

4. Inhalationsgerät nach Anspruch 1, 2 oder 3 dadurch gekennzeichnet, daß die mechanische Schalteinrichtung einen federvorgespannten Triggerkolben (15) im Luftkanal (9) aufweist, der mit einem Hebelarm eines Entriegelungshebels (14) verbunden ist, dessen anderer Hebelarm mit dem Auslösemechanismus (13, 19a) an der Vorspanneinrichtung der Pumpanordnung verbunden ist.

5. Inhalationsgerät nach einem der Ansprüche 1 bis 3, dadurch gekennzeichnet, daß die mechanische Schalteinrichtung eine flexible Membran (47) aufweist, deren eine Seite über einen Unterdruckkanal (41, 32a) mit dem Mundstück (11) und deren andere Seite mit der Umgebungsluft in Verbindung steht und der mechanische Auslöseelemente (49, 50) zugeordnet sind, die mit dem Auslösemechanismus (13, 19c, 46) an der Vorspanneinrichtung der Pumpanordnung in Wirkeingriff stehen.

6. Inhalationsgerät nach Anspruch 5, dadurch gekennzeichnet, daß der Luftkanal (9) im Mundstück (11) als Düse (39) ausgebildet ist, wobei der Bereich mit der höchsten Strömungsgeschwindigkeit im Bereich der Einmündung des Unterdruckkanals (41) angeordnet ist.

7. Inhalationsgerät nach Anspruch 5 oder 6, dadurch gekennzeichnet, daß ein Membranraum (48) vorgesehen ist, der auf der einen Seite durch die Membran (47) begrenzt ist und der einen Membrantopf (49) aufnimmt, dem Auslösestifte (50) zugeordnet sind, die beim Ansprechen der Membran und damit des Membrantopfes auf Unterdruck mit dem Auslösemechanismus (13, 19c, 46) an der Vorspanneinrichtung der Pumpanordnung in Wirkverbindung bringbar sind.

8. Inhalationsgerät nach einem der Ansprüche 1 bis 7, dadurch gekennzeichnet, daß die Dosiereinrichtung einen senkrecht zur Mundstückachse in Längsrichtung des Inhalationsgerätes angeordneten Dosierstempel (8) mit einer Dosierkammer (7) aufweist, die durch einen kerbförmigen Einschnitt mit einem inneren Siebboden (7a), der mit einer Luftzuführung (8a) in Verbindung steht, gebildet ist, und daß der Dosierstempel (8) kopfseitig von einem im Gehäuse (3) des Inhalationsgerätes in Längsrichtung verschiebbar gehalterten Druckknopf (1) aufgenommen ist, derart, daß bei manueller Betätigung des Druckknopfes (1) zunächst die Dosierkammer (7) befüllt, anschließend mit der offenen Seite vor die Düse (23) in dem Luftkanal (9) positioniert und mit ihrer Luftzuführung (8a) mit dem Luftaustritt (12a) der Pumpanordnung (12, 12a, 19, 20, 22) verbunden wird.

9. Inhalationsgerät nach einem der Ansprüche 1 bis 8, gekennzeichnet durch eine Dosiereinrichtung (8), bei der, zumindest ein Teil der Wandung (26, 26a) des Vorratsraumes (6) für die zu inhalierende pulverförmige Substanz (27) nachgebend ausgebildet ist, derart, daß er der Entleerung des Vorratsraumes im Rahmen der Befüllung der Dosierkammer (7) folgt.

10. Inhalationsgerät nach Anspruch 9, dadurch gekennzeichnet, daß die sich mit der Pulverschüttung (27) bewegende Wandung des Vorratsraumes (6) durch ein flexibles Band (26) gebildet ist, das in ein Magazin (25) eingeschlauft ist, das den Vorratsraum (6) auf Bandbreite begrenzt, und daß die Schlaufenöffnung durch einen die Dosiereinrichtung bildenden Dosierstift (8) mit mindestens einer Dosierkerbe (7) als Dosierkammer abgeschlossen ist, an dem die Schlaufenenden, den Dosierstift (8) umschlingend, vorbeigeführt sind.

11. Inhalationsgerät nach Anspruch 10, dadurch gekennzeichnet, daß das Band (26) aus einem flexiblen Material vorgegebener Zugfestigkeit und Oberflächenrauhigkeit besteht.

12. Inhalationsgerät nach Anspruch 10 oder 11, dadurch gekennzeichnet, daß das Band (26) am einen Ende der Schlaufe auf einer ersten Welle (Stift 33) fixiert ist und auf eine zweite Welle (Bandspannstift 34), die mit dem Dosierstift (8) mechanisch gekoppelt ist, aufwickelbar ist.

13. Inhalationsgerät nach Anspruch 12, dadurch gekennzeichnet, daß die Kopplung zwischen Dosierstift (8) und Bandspannstift (34) nach Art einer Rutschkupplung so getroffen ist, daß sie bei Übertragung eines Grenzdrehmomentes durchrutscht und somit die Kraft, mit der die Bandschlaufe die leicht verdichtete Pulverschüttung (27) gegen den Dosierstift (8) preßt, limitiert ist.

14. Inhalationsgerät nach Anspruch 13, dadurch gekennzeichnet, daß der Dosierstift (8) mit dem Bandspannstift (34) über einen Riemen (35) gekoppelt ist.

15. Inhalationsgerät nach einem der Ansprüche 10 bis 14, dadurch gekennzeichnet, daß die Schlaufenenden des Bandes (26) derart in Bezug auf den Dosierstift (8), diesen umschlingend, geführt sind, daß ihr gegenseitiger Abstand in Zugrichtung hinter dem Dosierstift kleiner als dessen Durchmesser ist (Umschlingungswinkel größer als 180°).

16. Inhalationsgerät nach Anspruch 2 und einem der Ansprüche 10 bis 15, dadurch gekennzeichnet, daß dem Dosierstift (8) die Dispergierdüse (29) im Magazin (25) konstruktiv, z.B. über eine Nocke, zugeordnet ist, derart, daß die Düse bei Drehung des Dosierstiftes im Bereich des Eintretens der Dosierkerbe (7) in den Düsenbereich leicht vom Dosierstift abhebt.

17. Inhalationsgerät nach einem der Ansprüche 10 bis 16, dadurch gekennzeichnet, daß das Magazin (25) zwei im Bandabstand angeordnete Scheiben als Gehäusestirnwände aufweist, in denen zentrisch der Dosierstift (8), diese drehbar durchdringend, sowie zwischen denen die Dispergierdüse (29) stationär angeordnet ist, und zwischen denen im Düsenabschnitt der Arretier- und Bandspannstift (33,34) aufgenommen ist, derart, daß ihr gegenseitiger innerer Umfangsabstand (X) kleiner als der Durchmesser des Dosierstiftes (8) ist.

## Claims

1. A propellant-free inhalation device, comprising a storage space (6) for a powdery medicinal substance to be inhaled, a manually operable metering device (8) being associated with said storage space for receiving a specified dosage of the medicinal substance for each inhalation process in at least one metering chamber (7), and comprising a lateral mouthpiece (11) for active inhalation which has an air channel (9) for distributing each dose of medicinal substance into the air stream, characterised in that a triggerable pump arrangement (12, 12a, 19, 20, 22), associated with the metering chamber, is provided, which has a manually operable pretensioning device (13, 19a; 19c) and a mechanical switching device (14 - 16; 47 - 50) that is in operative connection with the pretensioning device and the air channel (9) in the mouthpiece (11) such that the switching device responds to the reduced pressure produced upon inhalation and releases the pretensioning device, producing an extraneous air current which blows empty the filled metering chamber (7) and disperses the substance.

2. An inhalation device according to claim 1, characterised in that a dispersing nozzle (23; 29) is connected downstream of the metering chamber (7) through which the extraneous air current is guided.

3. An inhalation device according to claim 1 or 2, having a mechanical member (1, 42) for manual operation, characterised in that the member (1, 42) for manual operation of the metering device (8) is mechanically coupled with the actuating means of the pretensioning device (13, 19a; 19c).

4. An inhalation device according to claim 1, 2 or 3, characterised in that the mechanical switching device has a trigger piston (15) in the air channel (9) which is pretensioned by a spring and connected with one lever arm of a release lever (14), the other lever arm of which is connected with the release mechanism (13, 19a) provided on the pretensioning device of the pump arrangement.

5. An inhalation device according to any one of claims 1 to 3, characterised in that the mechanical switching device has a flexible membrane (47), one side of which is in communication with the mouthpiece (11), via a reduced pressure channel (41, 32a), and the other side of which is in communication with the surrounding air, mechanical release members (49, 50) being associated with the membrane, which are in an operative association with the release mechanism, (13, 19c, 46) provided on the pretensioning device of the pump arrangement.

6. An inhalation device according to claim 5, characterised in that the air channel (9) in the mouthpiece (11) is in the form of a nozzle (39), the region with the greatest velocity of flow being arranged in the region of the inlet of the reduced pressure channel (41).

7. An inhalation device according to claim 5 or 6, characterised in that a membrane chamber (48) is provided, one side of which is delimited by the membrane (47) and which accommodates a membrane cap (49) with which release pins (50) are associated which may be brought into an operative connection with the release mechanism (13, 19c, 46) provided on the pretensioning device of the pump arrangement, when the membrane and therefore the membrane cap respond to reduced pressure.

8. An inhalation device according to any one of claims 1 to 7, characterised in that the metering device has a metering piston (8) which is arranged in the longitudinal direction of the inhalation device at right angles to the axis of the mouthpiece, and which has a metering chamber (7) which is formed by a notch-shaped incision having an inner perforated bottom (7a) connected with an air inlet (8a), and that the top of the metering piston (8) is received by a push button (1) displaceably mounted in the longitudinal direction in the housing (3) of the inhalation device, so that firstly, upon manual actuation of the push button (1), the metering chamber (7) is filled, then placed in the air channel (9) with its open side in front of the nozzle (23) and connected, by means of its air inlet (8a), with the air outlet (12a) of the pump arrangement (12, 12a, 19, 20, 22).

9. An inhalation device according to any one of claims 1 to 8, characterised by a metering device (8), in which at least a part of the wall (26, 26a) of the storage space (6) for the powdery substance (27) is yieldingly constructed in such a way that emptying of the storage space ensues from the metering chamber (7) being filled.

10. An inhalation device according to claim 9, characterised in that the wall of the storage space (6), which moves as the powder (27) is filled, is formed by a flexible tape (26) looped into a container (25) which delimits the storage space (6) by means of the width of the tape, and that the loop opening is closed by means of a metering pin (8) which has at least one metering notch (7) as a metering chamber around which pin (8) the loop ends are wound.

11. An inhalation device according to claim 10, characterised in that the tape (26) is made of a flexible material with a specified tensile strength and surface roughness.

12. An inhalation device according to claim 10 or 11, characterised in that the tape (26) is secured at one end of the loop on a first shaft (pin 33) and may be wound onto a second shaft (tape tensioning pin 34) which is mechanically coupled to the metering pin (8).

13. An inhalation device according to claim 12, characterised in that the coupling between the metering pin (8) and the tape tensioning pin (34) is in the form of a slipping clutch so that it slips when a limiting torque is applied, thus limiting the force by means of which the tape loop presses the slightly compressed powder bulk (27) against the metering pin (8).

14. An inhalation device according to claim 13, characterised in that the metering pin (8) is coupled with the tape tensioning pin (34) via a belt (35).

15. An inhalation device according to any one of claims 10 to 14, characterised in that the loop ends of the tape (26) are guided with respect to the metering pin (8), while winding around it, so that their mutual spacing in the pulling direction behind the metering pin is smaller than the diameter of the pin (looping angle greater than 180°C).

16. An inhalation device according to claim 2 and any one of claims 10 to 15, characterised in that the dispersion nozzle (29) in the container (25) is constructionally associated with the metering pin (8), for example via a cam, so that the nozzle slightly lifts above the metering pin when the metering pin is rotated in the region of the entry of the metering notch (7), in the nozzle region.

17. An inhalation device according to any one of claims 10 to 16, characterised in that the container (25) has two plates as housing-end walls arranged at a spacing produced by the tape, in which the metering pin (8) is arranged centrally, the pin rotatably passing through the walls, and between which the dispersion nozzle (29) is fixedly arranged, and between which the locking and tape tensioning pins (33, 34) are received in the nozzle portion, so that their mutual inner circumferential spacing (X) is smaller than the diameter of the metering pin (8).

## Revendications

1. Inhalateur sans gaz propulseur avec une chambre de réserve (6) pour une substance médicinale en poudre, à inhaler, comprenant un dispositif de dosage (8) pouvant être actionné manuellement, pour l'admission d'une dose prédéterminée de la substance médicinale pour une inhalation, dans au moins une chambre de dosage (7), et présentant une embouchure (11) latérale pour l'aspiration active, laquelle présente un canal d'air (9) pour répartir la dose déterminée de la substance médicinale dans le courant d'air, caractérisé en ce qu'on prévoit un dispositif de pompe à déclenchement (12, 12a, 19, 20, 22), relié à la chambre de dosage (7), comprenant une installation de prétension (13, 19a; 19c) pouvant être actionnée manuellement et une installation de commande (14-16, 47-50) mécanique qui agit de concert avec l'installation de prétension et le canal d'air (9) de l'embouchure (11), de sorte que l'installation de commande dépend de la dépression produite par l'aspiration et que l'installation de prétension se déclenche par la production d'un courant d'air extérieur envahissant la chambre de dosage (7) chargée et dispersant la substance.

2. Inhalateur suivant la revendication 1, caractérisé en ce que la chambre de dosage (7) est placée après une tuyère de dispersion (23; 29) grâce à laquelle le courant d'air extérieur est guidé.

3. Inhalateur suivant la revendication 1 ou 2, comprenant un élément mécanique (1, 42) pour la mise en action manuelle, caractérisé en ce que l'élément (1, 42) pour la mise en action manuelle de l'installation de dosage (8) est couplé mécaniquement à l'activation de l'installation de prétension (13, 19a; 19c).

4. Inhalateur suivant la revendication 1, 2 ou 3, caractérisé en ce que l'installation de commande mécanique présente un piston de déclenchement (15) à ressort dans le canal d'air (9), auquel est relié un bras de levier d'un levier de déverrouillage (14); son autre bras de levier étant relié par un mécanisme de déclenchement (13, 19a) à l'installation de prétension du dispositif de pompe.

5. Inhalateur suivant l'une quelconque des revendications 1 à 3, caractérisé en ce que l'installation de commande mécanique présente une membrane flexible (47), un côté de celle-ci se trouvant en liaison avec l'embouchure (11) par un canal de dépression (41, 32a) et l'autre côté, en liaison avec l'air environnant et elle est reliée aux éléments de déclenchement (49, 50) mécaniques qui agissent avec le mécanisme de déclenchement (13, 19c, 46), sur l'installation de prétension du dispositif de pompe.

6. Inhalateur suivant la revendication 5, caractérisé en ce que la canal d'air (9) forme une tuyère (39) dans l'embouchure (11), où le domaine de débit le plus élevé est placé à proximité de l'embouchure du canal de dépression (41).

7. Inhalateur suivant la revendication 5 ou 6, caractérisé en ce qu'une chambre à membrane (48) est prévue, laquelle est limitée, d'un côté, par la membrane (47) et accueille un pot à membrane (49), qui sont raccordés au pivot de déclenchement (50) et qui peuvent être placés en relation de travail avec le mécanisme de déclenchement (13, 19c, 46) sur l'installation de prétension du dispositif de pompe, par correspondance de la membrane et ainsi, du pot à membrane, par dépression.

8. Inhalateur suivant l'une quelconque des revendications 1 à 7, caractérisé en ce que l'installation de dosage présente un piston de dosage (8) placé perpendiculairement à l'axe de la pièce d'embout et dans la direction longitudinale de l'inhalateur, avec une chambre de dosage (7), qui est constituée par une encoche avec un côté muni d'un tamis interne (7a), qui se trouve en liaison avec une adduction d'air (8a) et en ce que le piston de dosage (8) est raccordé, côté face, à un bouton poussoir (1) à positions multiples dans la direction longitudinale, dans le boîtier (3) de l'inhalateur, de sorte que, par mise en action manuelle du bouton poussoir (1), la chambre de dosage (7) se remplit, elle se place, ensuite, avec le côté ouvert devant la tuyère (23) du canal d'air (9) et est reliée par son adduction d'air (8a) à la sortie d'air (12a) du dispositif de pompe (12, 12a, 19, 20, 22).

9. Inhalateur suivant l'une quelconque des revendications 1 à 8, caractérisé par une installation de dosage (8), laquelle est formée par au moins une partie de la paroi (26, 26a) de la chambre de réserve (6) pour libérer la substance (27) poudreuse, à inhaler, de sorte que la vidange de la chambre de réserve se fasse par le remplissage de la chambre de dosage (7).

10. Inhalateur suivant la revendication 9, caractérisé en ce que la paroi de la chambre de réserve (6), se déplaçant avec le déchargement de poudre (27) est formée d'un ruban flexible (26), qui est inclus dans un magasin (25), qui limite la chambre de réserve (6) sur la largeur du ruban et en ce que l'ouverture coulissante est fermée par un pivot de dosage (8) formant une installation de dosage avec au moins une encoche de dosage (7) comme chambre de dosage, sur lequel les parties coulissantes défilent par le pivot de dosage (8) rotatif

11. Inhalateur suivant la revendication 10, caractérisé en ce que le ruban (26) consiste en une matière flexible d'une résistance et d'une rugosité de surface prédéterminées.

12. Inhalateur suivant la revendication 10 ou 11, caractérisé en ce que le ruban (26) est fixé par une extrémité de la partie coulissante à un premier arbre (pivot 33) et peut être enroulé à un deuxième arbre [pivot de tension du ruban (34)] qui est couplé mécaniquement au pivot de dosage (8).

13. Inhalateur suivant la revendication 12, caractérisé en ce que le couplage entre le pivot de dosage (8) et le pivot de tension du ruban (34) est réalisé, suivant la technique, par un couplage de glissement tel qu'il glisse par transmission d'un couple limité et ainsi, la force avec laquelle la partie coulissante du ruban presse le déchargement de poudre (27) facilement comprimé contre le pivot de dosage (8), est limitée.

14. Inhalateur suivant la revendication 13, caractérisé en ce que le pivot de dosage (8) est couplé au pivot de tension du ruban (34) par une courroie (35).

15. Inhalateur suivant l'une quelconque des revendications 10 à 14, caractérisé en ce que les parties coulissantes du ruban (26) sont guidées de telle façon qu'elles enlacent le pivot de dosage (8) et en ce que leur écartement, après le pivot de dosage, est inférieur à leur diamètre (magasin d'enlacement supérieur à 180°).

16. Inhalateur suivant la revendication 2 et l'une quelconque des revendications 10 à 15, caractérisé en ce que le pivot de dosage (8) est rattaché à la tuyère de dispersion (29) dans le magasin (25), par exemple par une rainure, de sorte que la tuyère se sépare facilement du pivot de dosage, par rotation du pivot de dosage dans la partie creuse de l'encoche de dosage (7).

17. Inhalateur suivant l'une quelconque des revendications 10 à 16, caractérisé en ce que le magasin (25) présente deux disques placés dans l'écart du ruban comme paroi du boîtier, dans lesquels le pivot de dosage (8), qui pénètre par rotation, est placé centralement ainsi qu'entre lesquels est placée la tuyère de dispersion (29), et entre lesquels, dans la section de la tuyère, sont placés les pivots de tension du ruban et d'arrêt (33, 34), de sorte que leur écart interne (X) est inférieur au diamètre du pivot de dosage (8).
